Europäisches Patentamt

European Patent Office

Office européen des brevets

(11) Publication number: **0 211 815**
**B1**

## (12) EUROPEAN PATENT SPECIFICATION

(45) Date of publication of patent specification: **18.04.90**

(51) Int. Cl.⁵: **A 61 F 13/15**

(21) Application number: **86850256.8**

(22) Date of filing: **15.07.86**

(54) **Disposable absorbent product such as a diaper, a sanitary towel or the like, and a method of producing such an article.**

(30) Priority: **31.07.85 JP 169634/85**

(43) Date of publication of application:
**25.02.87 Bulletin 87/09**

(45) Publication of the grant of the patent:
**18.04.90 Bulletin 90/16**

(84) Designated Contracting States:
**DE FR NL SE**

(56) References cited:
**EP-A-0 021 662**
**EP-A-0 059 015**
**DE-C-1 766 437**
**GB-A-2 098 871**
**US-A-3 527 221**
**US-A-4 331 501**

(73) Proprietor: **Mölnlycke AB**
**S-405 03 Göteborg (SE)**

(72) Inventor: **Ternström, Ingela**
**Persikovägen 13**
**S-43500 Mölnlycke (SE)**

(74) Representative: **Alfredson, Stig et al**
**H. ALBIHNS PATENTBYRA AB Box 3137**
**S-103 62 Stockholm (SE)**

Courier Press, Leamington Spa, England.

## Description

The present invention relates to a disposable absorbent product, e.g. a diaper, a sanitary towel or the like, comprising an absorption body, an outer layer of liquid-pervious material facing towards the wearer when the product is in use, and an outer layer impervious to liquid on the opposite side of the body, both these layers extending a distance past the contour of the absorption body, where they are mutually united, as well as being fixed to the body by a binding agent.

The invention also relates to a method of producing such a product.

In the systematic manufacture of disposable products of the kind mentioned, the sheath enveloping the absorption body is normally sealed with a binding agent. The outer skin or layer is furthermore attached to the absorption core, the object of attaching the liquid-pervious layer to the absorption core being to keep this layer in contact with the core for providing good liquid transport between layer and core, also for reinforcing the core, which normally comprises so-called fluffed cellulose, while the attachment between the liquid-impervious layer and core is made for reinforcing the latter.

Totally coating the layer with binding agent is unsuitable from several aspects. For manufacturing reasons, the binding agent used at present for disposable articles is hot-melt glue, and because of the cost it is inconceivable to coat the layers entirely. In addition, a completely covering coating of glue on the liquid-pervious skin is extremely unfavourable for liquid migration to the absorption body. In a known method of partially coating the layers with hot-melt glue, the latter is applied in transverse, separate glue beads. However, this has some substantial disadvantages. The beads of binding agent must namely be comparatively thick for a satisfactory attachment of the layers to take place. This naturally results in a large consumption of glue. The application of the glue in such beads further results in that the skin materials will be stiff, and thus uncomfortable to the wearer.

In another known method the glue is applied in separate dots. The stiffness problem is indeed avoided here, but that of large glue consumption still remains. As is also the case with transverse glue beads, the glue dots must namely be very thick for there to be secure mutual attachment of the layers' edge portions, which extend a distance past the edges of the absorption body. These end portions are namely united by compression, and for contact and adherence to take place in a satisfactory manner, it is important that the glue dots or beads applied to a layer have a thickness such that, when the layers are compressed, they are given the contact pressure necessary for adherence to take place.

An absorbent product of the kind described in the introduction has been provided by the present invention, and in relation to the attachment of the sheath, this product has a plurality of advantages compared with such products in the prior art.

A disposable product implemented in accordance with the invention is primarily characterized in that the layers have a coating of binding agent on their inner sides, which is so thinly applied that on application it has parted and formed a network-like pattern extending over the respective layer, and in that this binding coating is both the means for the mutual attachment of the two layers at the portions thereof extending past the contour of the absorption body and also the means of attachment between the respective layer and the absorption body.

An essential advantage in applying the binding agent in a network-like pattern over the insides of both layers is that a more secure attachment of the layers' edge portions along the contour of the absorption body is afforded. An attachment binding agent to binding agent namely takes place, which provides a secure attachment, even for low contact pressure.

The cohesive, network-like binding agent pattern on the insides of both layers also provides secure attachment of the layers to the absorption body, as well as better reinforcement thereof, compared with the previously known binding agent patterns described hereinbefore.

The inventive application of binding agent gives, in addition, considerably less consumption of the agent than applications involving beads or spots. For applications to both layers in accordance with the invention, there is namely a consumption of merely about 6 $g/m^2$ for each of the layers, whereas for beads or spots it is about 20 $g/m^2$.

The invention will now be described in detail with reference to an embodiment illustrated in the accompanying drawing, where

Figure 1 is a plan of an embodiment of an absorbent products implemented in accordance with the invention;

Figure 2 shows a cross-section of this product; and

Figure 3 schematically illustrates how the binding agent pattern is applied.

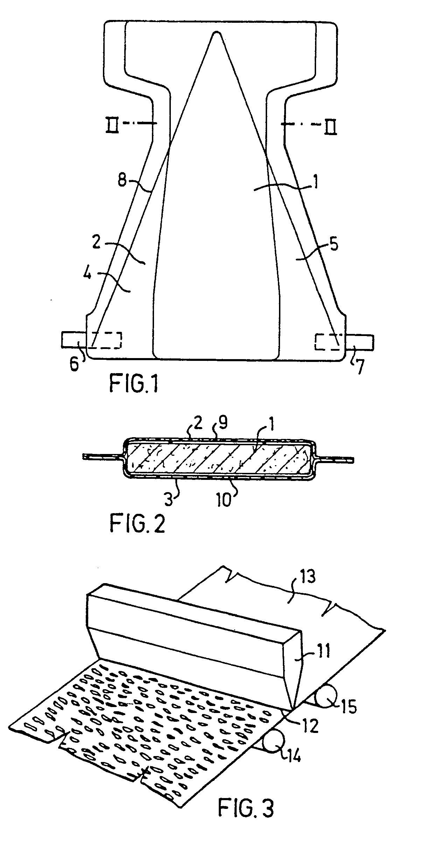

The product illustrated in Figure 1 comprises a diaper with a substantially T-shaped absorption body 1, suitably consisting of fluffed cellulose. On one side of the body there is placed a skin or layer 2 of liquid-pervious material, e.g. a fibre fabric, and on the other side of the body there is placed a skin or layer 3 of liquid-impervious material, e.g. polyethylene film. Both layers have portions extending past the absorption core, these portions being intended for mutual attachment. Starting from the crotch portion, and in a direction towards the back end of the diaper, the layers project increasingly outwards to form triangular flaps 4, 5, which are intended to go round the bottom of the wearer. The described diaper is a so-called complete disposable diaper, the triangular flaps 4, 5 of which are taken round the legs of the wearer and are fastened by ribbon flaps 6, 7 against the outside of the front end of the diaper, whereby a trouser-like unit is obtained.

The diaper is provided with elastic 8, which is arranged in a V-shaped pattern, with the tip of the pattern situated at the front end of the diaper.

The parts included in the diaper are kept together by a binding agent, which is applied to form a very thin, network-like pattern. The agent, which is applied to the insides of both layers 2, 3 has been denoted 9, 10 in Figure 2.

The principle for applying the binding agent in accordance with the invention is illustrated in Figure 3. The agent, which is in the form of a hot-melt glue, is supplied to a so-called slot nozzle 11 having a narrow outlet gap 12. The material web 13, which is to be coated with glue, is advanced past the nozzle and is urged into contact therewith by rolls 14, 15, the advancing web entraining glue from the nozzle. The amount of glue extracted from the nozzle is critical in forming the desired network-like pattern, and is controlled by the liquid pressure in the glue and the size of the nozzle. The latter should have a gap in the order of magnitude 0.3 mm.

With a suitable liquid pressure at the nozzle and the selection of a gap in the order of magnitude mentioned, the glue will part into strands on being drawn out of the nozzle by the material web and form a network-like pattern as illustrated in Figure 3.

With the aid of the hot-melt glue application, both the layers are attached to the absorption body, and the portions of the layers projecting past it are mutually attached. The elastic is mounted in a tensioned condition against one of the layers and is fastened thereto with the aid of the glue coating.

The invention is not restricted to the embodiment described here, since a plurality of modifications are possible within the scope of the following claims.

## Claims

1. Disposable, absorbent product, e.g. a diaper, a sanitary towel or the like, comprising an absorption body (1), an outer layer (2) of liquid-pervious material facing towards the wearer when in use, and an outer layer (3) impervious to liquid on the opposite side of the body, both these layers extending past the contour of the body, and being mutually united at the extended portions, and also attached to the body (1) by a binding agent, characterized in that the layers (2, 3) have a coating of binding agent (9, 10) on their inner sides, which is so thinly applied that on application it has parted and formed a network-like pattern extending over the respective layer, and in that this binding agent coating is both the means for the mutual attachment of the two layers at the portions thereof extending past the contour of the absorption body (1) and also the means of attachment between the respective layer and the absorption body.

2. Method of manufacturing a disposable product, e.g. a diaper, a sanitary towel or the like, said product including two outer layers (2, 3) which form a sheath enveloping an absorption body (1), both layers (2, 3) having such a size and being mounted in such a manner that they extend past the contour of the absorption body (1) and are mutually united at the extended portions, the method being characterized in that a binding agent coating (9, 10) is applied to the insides of both layers so thinly that the coatings part, on being applied, to form a network-like pattern of binding agent extending over the layers (2, 3) and in that the layers are attached to either side of the absorption body and are mutually attached at their portions extending past the contour of the absorption body with the aid of these binding agent coatings.

## Patentansprüche

1. Absorbierendes Wegwerferzeugnis, wie eine Windel, eine Monatsbinde oder dergleichen, mit einem Absorptionskörper (1), einer äußeren Schicht (2) aus flüssigkeitsdurchlässigem Werkstoff, der bei Gebrauch dem Träger zugewandt ist, und einer äußeren, für Flüssigkeiten undurchlässigen Schicht (3) an der entgegengesetzten Seite des Absorptionskörpers, wobei sich diese beiden Schichten über den Umriß des Absorptionskörpers hinaus erstrecken und an den verlängerten Abschnitten miteinander vereinigt sind, und ferner am Absorptionskörper (1) mittels eines Bindemittels befestigt sind, dadurch gekennzeichnet, daß die Schichten (2, 3) an ihren Innenseiten einen Überzug des Bindemittels (9, 10) aufweisen, das so dünn aufgetragen ist, daß es sich beim Auftrag zerteilt hat und ein netzwerkartiges Muster bildet, das über die jeweiligen Schichten verläuft, und daß dieser Überzug des Bindemittels sowohl ein Mittel für die gegenseitige Befestigung der beiden Schichten an deren Abschnitten ist, die sich über den Umriß des Absorptionskörpers hinaus erstrecken als auch das Befestigungsmittel zwischen der jeweiligen Schicht und dem Absorptionskörper.

2. Verfahren zur Herstellung eines Wegwerferzeugnisses wie eine Windel, eine Monatsbinde oder dergleichen, das zwei äußere Schichten (2, 3) aufweist, die eine Hülle bilden, die einen Absorptionskörper (1) umgibt, wobei beide Schichten (2, 3) eine solche Größe haben und derart angebracht sind, daß sie sich über den Umriß des Absorptionskörpers (1) hinaus erstrecken und an den verlängerten Abschnitten miteinander verbunden sind, dadurch gekennzeichnet, daß ein Überzug eines Bindemittels (9, 10) auf die Innenseiten beider Schichten so dünn aufgebracht wird, daß der Überzugsteil beim Auftrag ein netzwerkartiges Muster des Bindemittels bildet, das sich über die Schichten (2, 3) erstreckt und das die Schichten an jeder Seite des Absorptionskörpers befestigt werden und an ihren Abschnitten, die sich über den Umriß des Absorptionskörpers hinaus erstrecken, mit Hilfe dieser Überzüge des Bindemittels miteinander verbunden werden.

**Revendications**

1. Produit jetable absorbant, par exemple couche-culotte, serviette hygiénique ou analogue, comportant un corps absorbant (1), une couche extérieure (2) en un matériau perméable aux liquides faisant face à la personne lors de l'utilisation, et une couche extérieure (3) imperméable aux liquides sur la face opposée du corps, ces deux couches s'étendant autour du contour du corps, et étant unies l'une à l'autre sur des parties étendues, et étant également fixées au corps (1) par un agent adhésif, caractérisé en ce que les couches (2, 3) présentent un revêtement d'agent adhésif (9, 10) sur leur face intérieure, qui est appliqué de manière tellement mince que, lors de l'application, il se divise et forme un motif en réseau s'étendant sur la couche respective, et en ce que ce revêtement d'agent adhésif constitue tout à la fois le moyen de fixation mutuelle des deux couches aux parties de celles-ci, s'étendant au-delà du contour du corps absorbant (1) et, également, le moyen de fixation entre la couche respective et le corps absorbant.

2. Procédé de fabrication d'un produit jetable, par exemple une couche-culotte, une serviette hygiénique ou analogue, ledit produit comportant deux couches extérieures (2, 3) qui forment un étui enveloppant un corps absorbant (1), les deux couches (2, 3) étant d'une taille telle et étant montées d'une façon telle qu'elles s'étendant au-delà du contour du corps absorbant (1) et qu'elles soient unies l'une à l'autre sur les parties étendues, le procédé étant caractérisé en ce qu'un revêtement d'agent adhésif (9, 10) est appliqué sur la face interne des deux couches, de manière tellement mince que les revêtements se divisent, lors de l'application, de façon à former un motif en réseau d'agent adhésif s'étendant sur les couches (2, 3) et en ce que les couches sont fixées aux deux côtés du corps absorbant et sont mutuellement fixées sur leurs parties s'étendant au-delà du contour du corps absorbant à l'aide de ces revêtements d'agent adhésif.

FIG.1

FIG. 2

FIG. 3